# EUROPEAN PATENT SPECIFICATION

(11) **EP 4 139 323 B1**
(45) Date of publication and mention of the grant of the patent: **20.08.2025**
(21) Application number: 21719148.5
(22) Date of filing: 19.04.2021
(51) Int. Cl.: C07J 9/00, C07J 41/00

(54) **PREPARATION OF 7-DEHYDROCHOLESTEROL USING SPECIFIC AROMATIC SOLVENTS**
HERSTELLUNG VON 7-DEHYDROCHOLESTERIN UNTER VERWENDUNG BESTIMMTER AROMATISCHER LÖSUNGSMITTEL
PRÉPARATION DE 7-DÉHYDROCHOLESTÉROL À L'AIDE DE SOLVANTS AROMATIQUES SPÉCIFIQUES

(30) Priority: 23.04.2020 EP 20171011
(43) Date of publication of application: 01.03.2023
(73) Proprietor: DSM IP Assets B.V., 6221 BE Maastricht (NL)
(72) Inventor: FURER, Claude Alban, 4303 Kaiseraugst (CH); MARTY, Maurus, 4303 Kaiseraugst (CH); SCHUETZ, Jan, 4303 Kaiseraugst (CH)
(74) Representative: DSM IP ANH
(86) International application number: PCT/EP2021/060102
(87) International publication number: WO 2021/213984

(56) References cited:
- CN-A- 101 381 389
- CN-A- 105 646 632
- CN-A- 109 761 867
- E V YABLONSKAYA ET AL: "Synthesis of 7-dehydrocholesterol acetate", CHEMISTRY OF NATURAL COMPOUNDS, vol. 9, 1 January 1973 (1973-01-01), pages 708 - 709, XP055737754

## Description

### Technical Field

The present invention relates to the synthesis of 7-dehydrocholesterol or its OH-protected forms, respectively.

### Background of the invention

7-Dehydrocholesterol is a key intermediate used in the synthesis for cholecalciferol (=vitamin D3).

A possible pathway of synthesis of 7-dehydrocholesterol is disclosed in CN 101381389 starting from cholesteryl acetate and using an intermediate 7-tosylhydrazone cholesterylacetate in an organic solvent and a base. Similar processes are also disclosed in CN-A-109 761 867 and Chemistry of Natural Compounds Vol 9, pp 708-709 (1973). It has been found that this procedure has significant drawbacks.

### Summary of the invention

In a newly developed process for the synthesis of 7-dehydrocholesterol, it has been observed that a higher molecular compound is formed as a byproduct.

It has been also found that this higher molecular compound when present in high amounts is deposited on the surface of the photoreactor in the photoreaction of 7-dehydrocholesterol of at a later stage in the synthesis of vitamin D3. This is of course very disadvantageous. At lower concentrations, however, this is no longer the case.

It has now be found that the process according to claim 1 solves surprisingly this problem. A significant lower amount of the new higher molecular compound is formed. This process enables to reduce the amount of this higher molecular compound to the range of between 0.00001% and 0.7%, particular between 0.00001% and 0.5%, in relation to 7-dehydrocholesterol.

Furthermore, it has been found that the new higher molecular species which has been identified and its structure characterized, is coloured and can be used as a new colorant. However, this does not form part of the present invention.

It has been also found that the sequence of steps is very crucial for the present invention. In a first step the salt of the intermediate OH-protected 7-tosylhydrazone cholesterol (formula (II)) is added to an already hot solvent (> 60°C) of formula (III). In comparing this process to a process in which a cold (< 50°C, particularly room temperature) mixture of solvent and the intermediate OH-protected 7-tosylhydrazone cholesterol (formula (II)), is heated up subsequently, we could show that the amount of the observed higher molecular compound is significantly lower and high conversion and yield can be obtained.

Further aspects of the invention are subject of further independent claims. Particularly preferred embodiments are subject of dependent claims.

### Detailed description of the invention

In a first aspect the present invention relates to a process of manufacturing a compound of the formula (I) 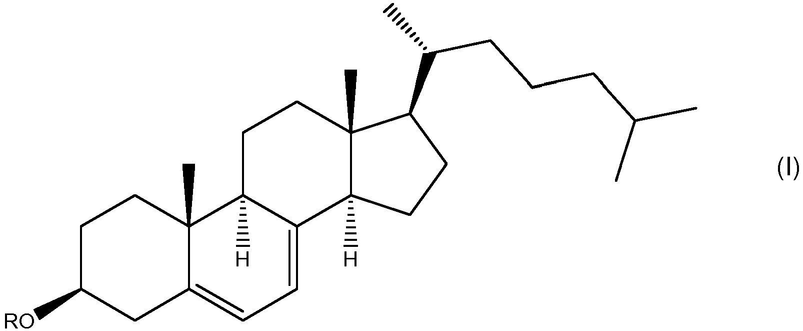 comprising the step
a) adding a salt of the formula (II) to a solvent of the formula (III) at a temperature of between 60 °C and the boiling point of said solvent 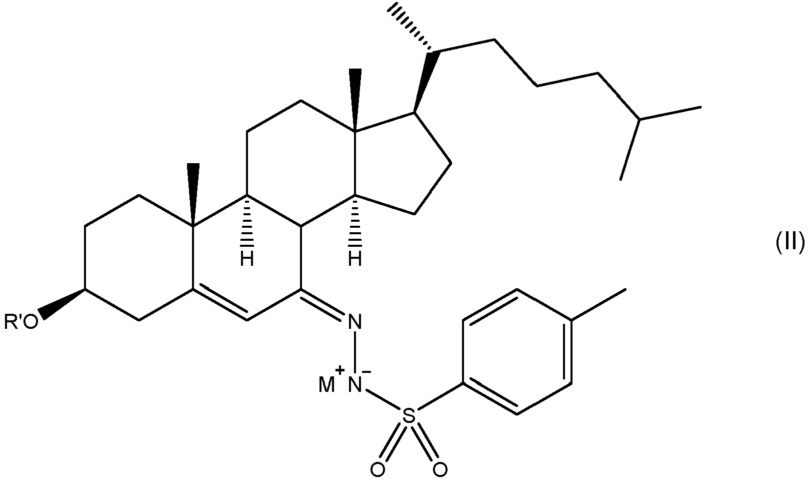 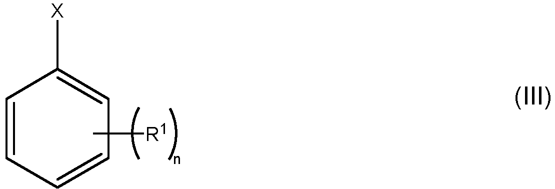 characterized in that
   R' represents an OH-protecting group;
   R represents H or R';
   M is an alkali metal atom, preferably Li;
   X represents either a halogen atom, preferably Cl, or a C₁₋₆-alkoxy group;
   R¹ represents an C₁₋₆-alkyl group;
   n = 0 or 1 or 2 or 3, preferably 0;
   with the proviso, that if R represents H,
      the process comprises further a step z) which takes place after step a) z) deprotecting the protected hydroxyl group of the compound of the formula (I-A) 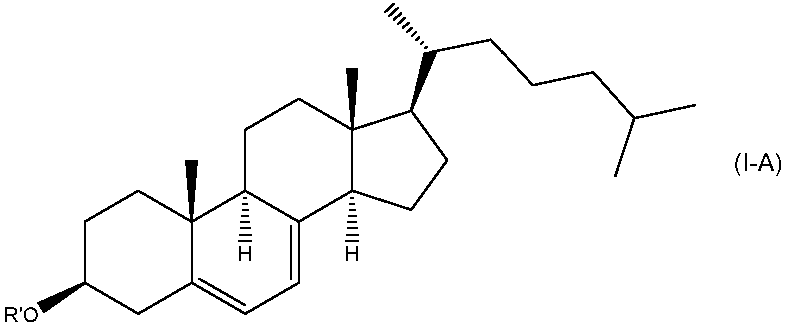
   to form the compound of the formula (I).

For sake of clarity, some terms as used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group. Hence, "propyl" can be "n-propyl" or "iso-propyl" (=isopropyl). Analogously, "butyl" can be "n-butyl" or "iso-butyl" or "sec-butyl" or "tert-butyl".

**In** the present document, "aralkyl" group is an alkyl group of which at least one H is substituted by an aryl group. Hence, for example, benzyl (=C₆H₅-CH₂-) is a C₇-aralkyl group.

**In** the present document, "alkylaryl" group is an aryl group of which at least one H of the aromatic ring is substituted by an alkyl group. Hence, for example, ethylphenyl (CH₃-CH₂-C₆H₄-) is a C₈-alkylaryl group.

**In** case identical labels for symbols or groups are present in several formulae, in the present document, the definition of said group or symbol made in the context of one specific formula applies also to other formulae which comprises the same said label.

R' represents an OH-protecting group. An OH-protecting group is a group which protects a hydroxyl group and the protecting group can be easily removed, i.e. by state-of-the-art methods, resulting to the respective compound with the free alcoholic group again.

The OH-protecting group R' is introduced by a chemical reaction of the compound of the respective formula having H instead of the OH-protecting group with a protecting agent.

The protecting agents leading to the corresponding OH-protecting groups are known to the person skilled in the art, as well as the chemical process and conditions for this reaction. If, for example, the OH-protecting group forms with the rest of the molecule an ester, the suitable protecting agent is for example an acid, an anhydride, or an acyl halide.

The OH-protecting group R' is particularly selected from the groups consisting of 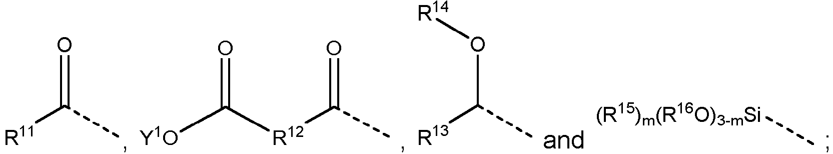
wherein R¹¹ represents H, a C₁₋₁₅-alkyl or a fluorinated C₁₋₁₅-alkyl or a C₁₋₁₅-cycloalkyl or a C₇₋₁₅-aralkyl group or a C₇₋₁₀-arylalkyl group or a phenyl group; R¹² represents a C₁₋₁₅-alkylene or a C₆₋₁₅-alkylene group;
and wherein either
   R¹³ represents a C₁₋₁₅-alkyl group or an alkyleneoxyalkyl group or a polyoxyalkylene group;
   R¹⁴ represents hydrogen or a C₁₋₁₅-alkyl group;
      or
   R¹³ and R¹⁴ represent together a C₃₋₇-alkylene group forming a 5 to 7 membered ring;
and wherein R¹⁵ represents a C₁₋₁₅-alkyl group and R¹⁶ represents a C₁₋₄-alkyl group and m = 0 or 1 or 2 or 3, preferably m=3;
and wherein the single dotted line represents the bond by which said substituent is bound to the rest of a molecule.

If R' is represented by 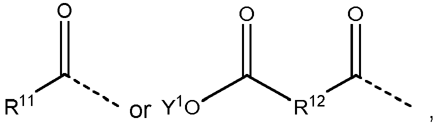 the respective compound is an ester of a carboxylic acid or dicarboxylic acid, which can be formed by the reaction of the respective protecting agent with the hydroxyl group. In this case, the protecting agent may be for example an anhydride or halide of the respective carboxylic acid (1) or dicarboxylic acid (2).

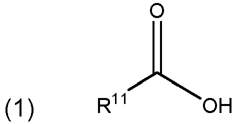

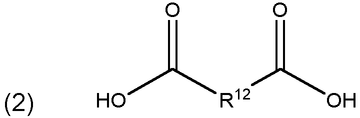

If the compound of the respective formula is an ester of a carboxylic acid or dicarboxylic acid, it is preferred that R' is an C₁₋₇-acyl, preferably acetyl, trifluoro acetyl, propionyl or benzoyl group, or a substituted benzoyl group.

Esters can be easily deprotected under the influence of an acid or a base.

If R' is 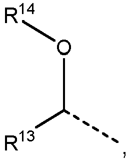 the respective compound is an acetal, which can be formed by the reaction of the respective protecting agent with the hydroxyl group. In this case, the protecting agent may be for example, a respective aldehyde, alkyl halide, e.g. MeO(CH₂)₂OCH₂Cl, or an enol ether, e.g. 3,4-dihydro-2*H*-pyran.

In this case, the substituent R' is preferably 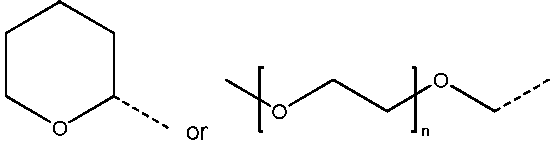 with n=0 or 1.

In some instances, acetals are also called "ethers", particularly in the cases mentioned above: methoxymethyl ether (MOM-ether), β-methoxyethoxymethyl ether (MEM-ether) or tetrahydropyranyl ether (THP-ether).

Acetals can be easily deprotected under the influence of acids.

In another preferred embodiment, the respective compound is an ester of phosphoric acid, pyrophosphoric acid, phosphorous acid, sulphuric acid or sulphurous acid.

Depending on the reaction conditions, the esterification is either complete or partial, leaving some residual acid groups of the respective acid non-esterified.

It is most preferred that the protecting group R' is a benzoyl group or a C₁₋₄-acyl group, particularly acetyl or trifluoro acetyl group, more particularly acetyl group. The molecules in which R' represents an acyl group, particularly an acetyl group, can be easily prepared from the corresponding unprotected molecule by esterification, and the unprotected alcohol can be obtained from the corresponding ester by ester hydrolysis, particular by strong bases such as NaOH or KOH.

It is preferred that the protecting group R' represents an acyl group, preferably an acetyl or benzoyl group, particularly an acetyl group:

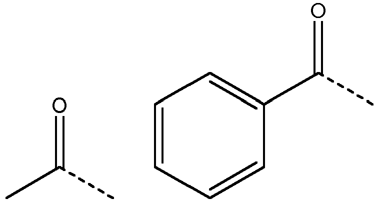

In one embodiment R represents a R', in other words, R represents an OH-protecting group.

In the other embodiment R represents H. In this case, the compound of formula (I-A) resulting from step a), having a protected OH group, needs to undergo in a further step z) a deprotection reaction to deprotect the protected OH group to form a free hydroxyl group, and, hence, compound of formula (I) having a free hydroxy group (R'=H).

The deprotection of the protected hydroxyl group is known by the person skilled in the art, and is particularly performed as mentioned above in the discussion of the specific protecting groups.

**It** is preferred that the compound of the formula (II) is prepared by salt formation of a compound of the formula (IV) 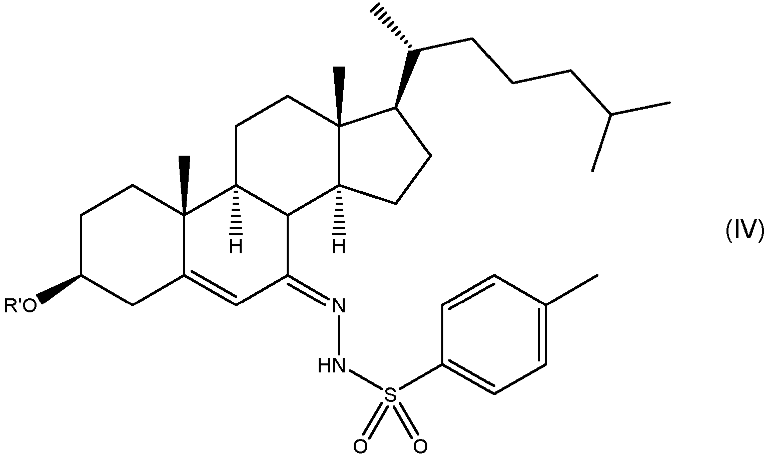 and a strong base comprising an alkali metal, particularly an alkali metal alcoholate, amide, hydride or complex hydride or a alkali metal alkyl, particularly butyllithium, preferably by NaNH₂ or LiNH₂.

The compound of formula (IV) is readily available from tosylhydrazide and the compound of formula (IV-a) which itself can be obtained from compound (IV-b):

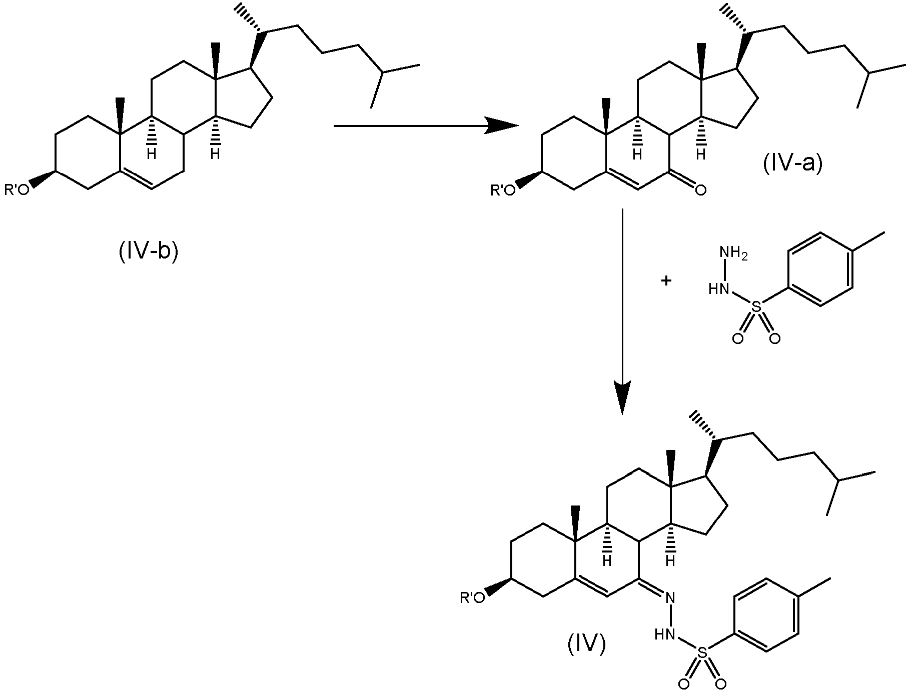

The alkali metal amide is preferably obtained by in situ formation from a mixture of the respective alkali metal and ammonia, particularly in the presence of an iron catalyst.

The formation of a salt of the formula (II) is preferably made in a solvent of formula(III).

Preferably, the base is suspended in the solvent of formula (III) is mixed with compound of formula (IV) at a temperature preferably of lower than 50°C, preferably between 10 and 45°C, most preferably at around room temperature, forming the salt in a dispersion.

It is preferred that the amount of solvent of formula (III) in this dispersion is less than 35%, preferably less than 30 % of the solvent of formula (III) used in step a).

Principally, the solvent can be removed and the salt can be isolated and added in step a) as solid salt or the salt can be added in step a) as said dispersion. The base is preferably used in a stoichiometric excess relative to compound of formula (IV). Typically the molar ratio of base/compound of formula (IV) is between 1.5: 1 and 10:1, more preferably between 2:1 and 4:1.

In step a) the solvent of formula (III) is heated to a temperature of between 60 °C and the boiling point of said solvent. To said warm solvent the salt of formula (II) is then added, preferably under stirring. It has been found that it is preferably that the salt is added slowly.

Hence, the salt of formula (II) is preferably added over a time range of between 15 and 60 minutes, preferably between 25 and 40 minutes.

It is preferred that the multiplication product of temperature of the solvent (in K) and the time of adding (in min) is between 9'000 and 20'000 Kmin, preferably between 11'000 and 14'000 Kmin.

It is preferred that after the salt is completely added, the mixture is continued to be stirred for a time of preferably between 30 minutes and 8 hours, more preferably between 2 and 5 hours.

It is preferred that the salt of the formula (II) is added in step a) during a time period of between 1 and 120 minutes, preferably between 10 and 60 minutes.

The reaction of step a) is performed using a solvent of formula (III)

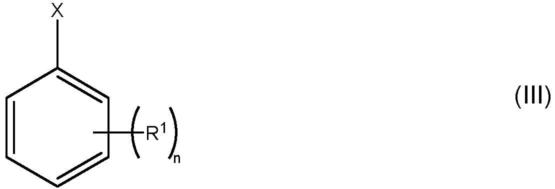

R¹ represents an C₁₋₆-alkyl group and n = 0 or 1 or 2 or 3, preferably 0.

Furthermore, X represents either a halogen atom, preferably Cl, or a C₁₋₆-alkoxy group.

In a first embodiment, X represents a C₁₋₆-alkoxy group. The methoxy group A is the preferred C₁₋₆ alkoxy group.

In this embodiment, the solvent of formula (III) is preferably selected from the group consisting of anisole, 2-methylanisiole, 3-methylanisole, and 4-methylanisole, preferably anisole.

In another, and preferred, embodiment X represents a halogen atom, preferably Cl.

Hence, compound of formula (III) can have up to 3 C₁₋₆-alkyl groups bound directly on the aromatic ring. The solvent of formula (III) is preferably selected from the group consisting of chlorobenzene, o-chlorotoluene, m-chlorotoluene, p-chlorotoluene. Most preferred, the solvent of formula (III) is chlorobenzene.

It has been found that when the reaction is performed at higher temperatures, the yield can be increased substantially.

It is preferred that the temperature in step a) is between 60° and reflux temperature of said solvent at ambient pressure, preferably between 80°C and 132°C.

The reaction of step a) can be performed particularly at ambient pressure or under pressure.

In case the reaction of step a) is performed under pressure, it is preferred that the process is performed under a pressure of larger than 1 bar.

In this case, it is preferred that the temperature of the solvent is between 60° and 200°C.

In step a), compound of formula (V) is formed at very low amounts. Compound of formula (V) has a significantly higher molecular weight as compared to compound of formula (I) respectively (II).

The structure of the new compound of formula (V) has been fully identified and the compound is characterized particularly by ¹H-NMR, ¹³C-NMR and MS.

Compound of formula (V) (R=OH):
¹H-NMR (300 MHz, CDCl₃): δ (ppm) = 0.72 (s, 6H), 0.86 (d, 6H, J=1.32 Hz), 0.88 (d, 6H, J=1.32 Hz), 0.94 (d, 6H, J=6.40 Hz), 1.01 - 1.63 (m, 42H), 1.80 (s, 2H), 1.90 (m, 6H), 2.04 (d, 2H, J = 12.62 Hz), 2.38 (m, 6H) 2.56 (m, 2H), 3.63 (m, 2H), 6.37 (s, 2H);
¹³C-NMR (75 MHz, CDCl₃): δ (ppm) = 12.3,17.9, 19.0, 21.0, 22.6, 22.9, 23.8, 24.6, 27.4, 28.0, 31.5, 35.7, 36.3, 36.6, 38.5, 38.6, 39.6, 39.8, 42.5, 43.0, 49.6, 50.5, 54.7, 71.2, 117.4, 153.5, 161.2.
LC-MS (ES) m/z: 797 [M⁺], 780 [M⁺+H-H₂O], 382 [monomer of M⁺+H-H₂O].

The compounds having protected groups (such as acetate) could been identified accordingly as well.

Disclosed herein, but not forming part of the present invention, is the compound of formula (V)

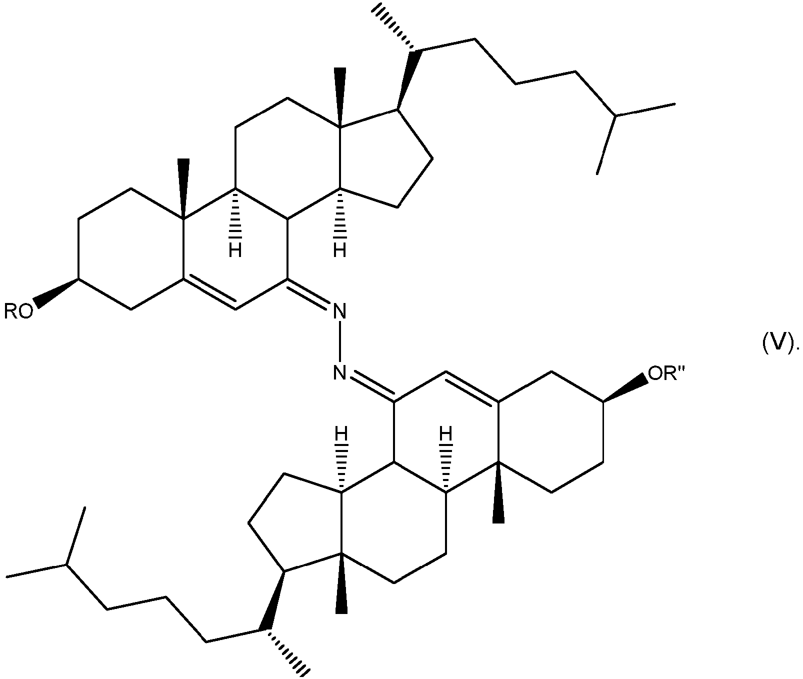

The residues R and R" represents either H or an OH-protecting group.

The OH-protecting group is as described already before for the compound of formula (I) or (II).

The OH-protecting group is preferably an acyl group, preferably an acetyl group.

It is further preferred that R=R".

The compound of formula (V) is yellow and can be used as a new colorant. However, this does not form part of the present invention.

As mentioned above the compound of formula (V) is formed at very low amounts parallel to the formation of compound (I).

It has been found that the process leads quantitatively, i.e. in high conversion and yield to the 7-dehydrocholesterol or its OH-protected forms, respectively.

It has been further found by the process of the invention the amount of compound of the formula (V) can be even further reduced, particularly when working at higher dilution and at higher temperature of solvent of formula (III).

Therefore, disclosed herein, but not forming part of the present invention, is a composition comprising
i) compound of the formula (I); and
ii) compound of the formula (V) 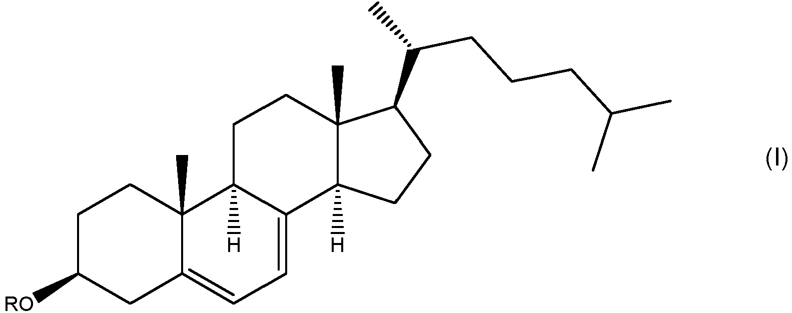 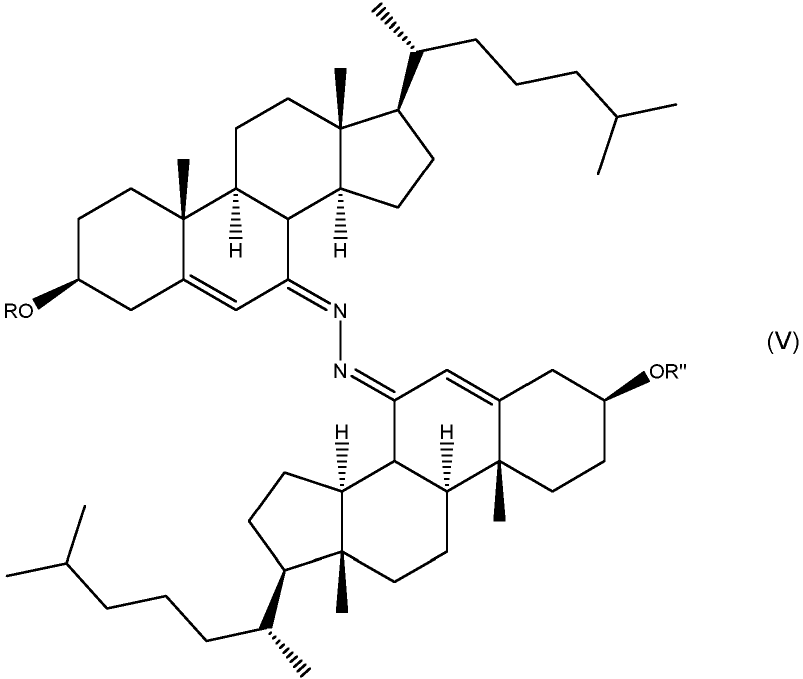 wherein
   R represents H or an OH-protecting group;
   characterized in that the ratio ***w_{V}*/*w_{I}*** in the composition is between 0.00001% and 0.7%, preferably between 0.00001% and 0.6%, particularly between 0.00001% and 0.5%, characterized in that ***w_{I}*** is the weight of the compound of the formula (I) and ***w_{V}*** is the weight of the compound of the formula (V).

Details for the OH-group have already been given before.

### Examples

The present invention is further illustrated by the following experiments.

### Synthesis of 7-dehydrocholesterol acetate (compound of salt of formula(I), R= R'=CH₃COO) (Process I)(comparison)

2.972 equivalents of LiNH₂ have been added to chlorobenzene (amount as to form a concentration, relative to 7-tosylhydrazone cholesterylacetate, as given in table 1) and stirred. Then 1.0 equivalent of 7-tosylhydrazone cholesterylacetate (=Compound of formula (IV), R'=CH₃COO) has been added at 23°C under stirring during 30 minutes, forming a dispersion of the Li salt (=compound of formula (II), M=Li, R'=CH₃COO)).

Then the dispersion has been stirred for the time (*tᵣ*) at a temperature (*T_{R}*) given in table 1). Then the mixture has been filtered. The filtrate was extracted twice with water and once with saturated NaCl solution. After evaporation of the chlorobenzene the product has been isolated in the yield given in table 1. The amount of the compound of formula (V) has been determined and is given in table 1.

### Synthesis of salt of formula(II)

2.972 equivalents of LiNH₂ have been added to 22% or 27%, respectively, of the total amount of chlorobenzene (for obtaining in process II a concentration of 7-tosylhydrazone cholesterylacetate in chlorobenzene of 0.11 or 0.09 g/ml, respectively)(see conc. in table 1) and stirred. Then 1.0 equivalent of 7-tosylhydrazone cholesterylacetate (=Compound of formula (IV), R'=CH₃COO) has been added at 23°C under stirring during 30 minutes, forming a dispersion of the Li salt (=compound of formula (II), M=Li, R'=CH₃COO) (***LiSalt***).

### Synthesis of 7-dehydrocholesterol acetate (compound of salt of formula(I), R= R'=CH₃COO) (Process II)

The rest (i.e. 78% or 73%, respectively) of the total amount of chlorobenzene has been separately heated up to the temperature (*T_{CB}*). The dispersion of the above Li salt (***LiSalt***) has been added under stirring for the time (*t_{add}*) given in table 1 to said hot chlorobenzene. The reaction mixture was stirred for the time (*tᵣ*) at a temperature (*T_{R}*) given in table 1. Then the mixture has been filtered. The filtrate was extracted twice with water and once with saturated NaCl solution. After evaporation of the chlorobenzene the product has been isolated in the yield given in table 1. The amount of the compound of formula (V) has been determined and is given in table 1.

**Table 1.Results of experiments using process II vs. process I.**

| | ***Ref.1*** | ***1*** | ***2*** | ***3*** | ***4*** | ***5*** | ***6*** |
|---|---|---|---|---|---|---|---|
| Process | I | II | II | II | II | II | II |
| Conc.[g/ml]* | 0.11 | 0.11 | 0.11 | 0.09 | 0.09 | 0.09 | 0.09 |
| *T_{CB}* [°C] | 25 | 100 | 100 | 100 | 100 | 100 | 132 |
| *T_{CB}* [K] | 298 | 373 | 373 | 373 | 373 | 373 | 405 |
| *t_{ad}* [min] | 30 | 30 | 1 | 30 | 30 | 25 | 30 |
| *T_{R}* [°C] | 100 | 100 | 100 | 100 | 100 | 100 | 132 |
| *tr* [h] | 4 | 4 | 4 | 3 | 4 | 4.5 | 4 |
| *T_{CB}*t_{ad}*[Kmin] | 8940 | 11190 | 373 | 11190 | 11190 | 9325 | 12150 |
| Conversion [%] | 97 | 97 | 97 | 95 | 96 | 100 | 98 |
| Yield** [%] | 81 | 81 | 76 | 75 | 73 | 54 | 86 |
| (V)/(I)[%]*** | 0.799 | 0.544 | 0.685 | 0.467 | 0.448 | 0.477 | 0.118 |

| | | | | | | | |
|---|---|---|---|---|---|---|---|
| *concentration of 7-tosylhydrazone cholesterylacetate in chlorobenzene **of compound of formula (I) ***weight ratio of compound of formula (V) to compound of formula (I) | | | | | | | |

In both processes the identity of compound has been verified by NMR and MS (see before) and the amount of the individual compounds has been quantified by HPLC.

In process II the salt of the formula (II) is added to hot chlorobenzene compound whereas in the process (II) (comparison) the salt of the formula (II) is prepared in situ and directly to the reaction temperature. The comparison of examples ***Ref.1*** and ***1*** show that the process (I) leads to significant reduction of compound of formula (V) whereas conversion and yield remain the same. The comparison of examples ***1*** and ***2*** shows that it is beneficial to add the salt of formula (II) slowly, i.e. preferably within a time of more than 10 minutes. The comparison of example ***1*** and ***4*** shows that by working in a lower concentration the amount of compound of formula (V) is reduced, that the yield in compound of formula (I), however, slightly drops. Comparison of Example ***6*** with examples ***1*** respectively ***4*** finally shows that it is advantageous to increase the temperature of chlorobenzene higher, to its boiling boing (reflux conditions) increases the yield significantly and have even further reduction in the amount of compound of formula (V) and that the slight drop in yield found in working at lower concentration can be compensated.

### Synthesis of 7-dehydrocholesterol (compound of salt of formula(I), R=H

7-dehydrocholesterol acetate as formed above (Examples ***1-6*)** are saponified by KOH and subsequent neutralization using acetic acid to yield 7-dehydrocholesterol, which can be quantitatively isolated.

## Claims

1. Process of manufacturing a compound of the formula (I) 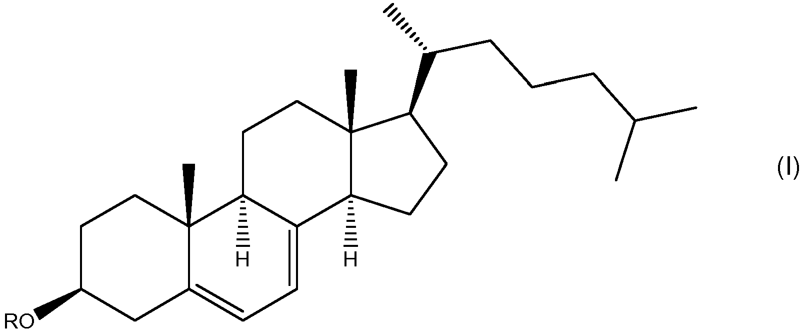 comprising the step
a) adding a salt of the formula (II) to a solvent of the formula (III) at a temperature of between 60 °C and the boiling point of said solvent 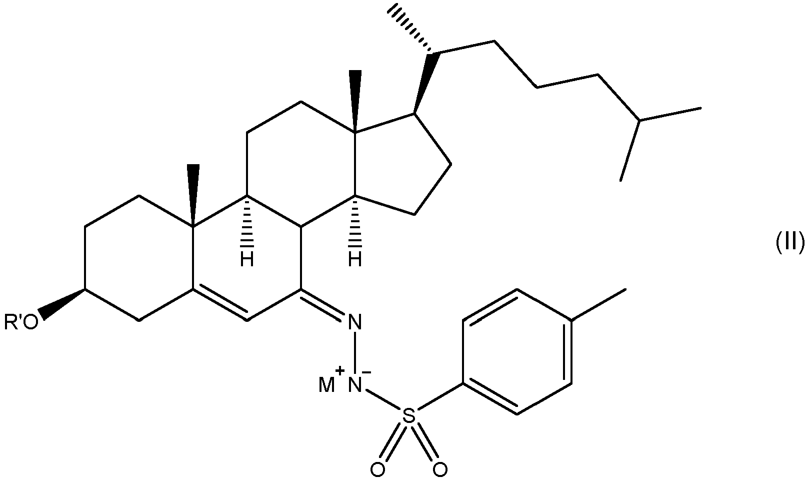 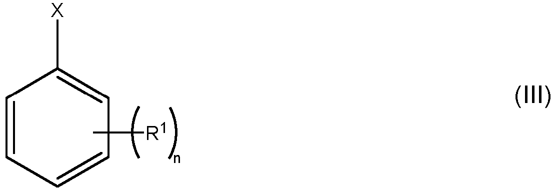 **characterized in that**
R' represents an OH-protecting group;
R represents H or R';
M is an alkali metal atom, preferably Li;
X represents either a halogen atom, preferably Cl, or a C₁₋₆-alkoxy group; R¹ represents an C₁₋₆-alkyl group;
n = 0 or 1 or 2 or 3, preferably 0;
with the proviso, that if R represents H,
the process comprises further a step z) which takes place after step a) z) deprotecting the protected hydroxyl group of the compound of the formula (I-A) 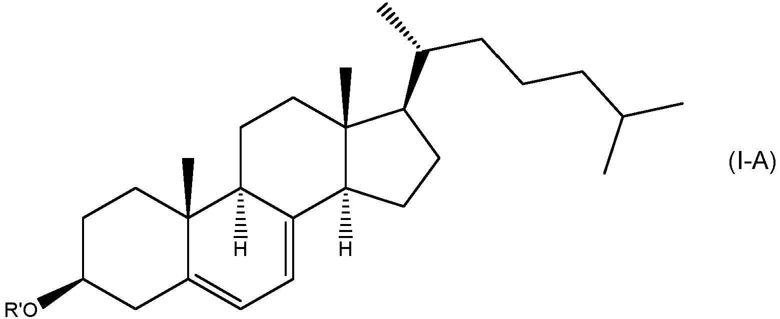
to form the compound of the formula (I).

2. The process according to claim 2 **characterized in that** the compound of the formula (II) is prepared by salt formation of a compound of the formula (IV) 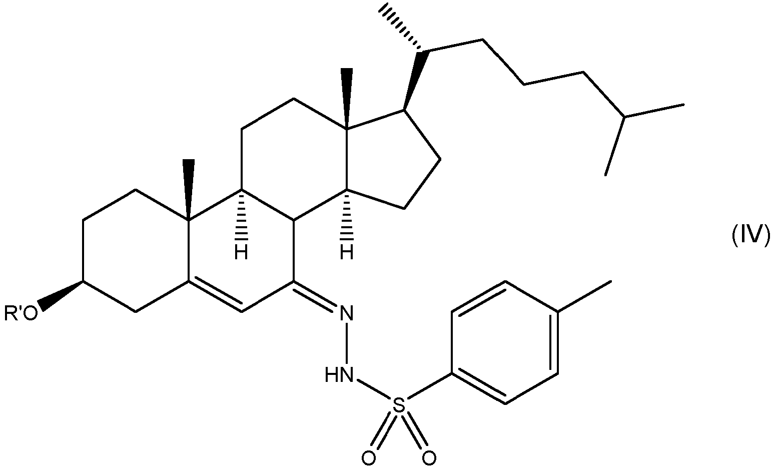 and a strong base comprising an alkali metal, particularly an alkali metal alcoholate, amide, hydride or complex hydride, preferably by NaNH₂ or LiNH₂.

3. The process according to claim 1 or 2 **characterized in that** the salt of the formula (II) is added in step a) during a time period of between 1 and 120 minutes, preferably between 10 and 60 minutes.

4. The process according to any of the preceding claims **characterized in that** the temperature of the solvent is between 60° and reflux temperature of said solvent at ambient pressure, preferably between 80°C and 132°C.

5. The process according to any of the preceding claims 1-3 **characterized in that** the process is performed under a pressure of larger than 1 bar.

6. The process according to claim 6 **characterized in that** the temperature of the solvent is between 60° and 200°C.

7. The process according to any of the preceding claims **characterized in that** R' represents an acyl group, preferably an acetyl or benzoyl group, particularly an acetyl group.

## Patentansprüche

1. Verfahren zur Herstellung einer Verbindung der Formel (I) 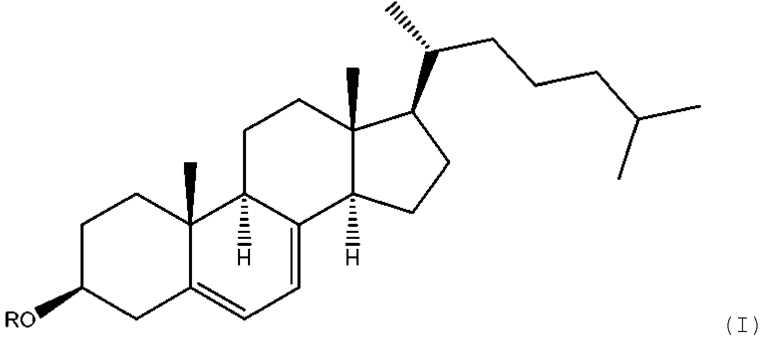 umfassend den Schritt
a) Zugeben eines Salzes der Formel (II) zu einem Lösungsmittel der Formel (III) bei einer Temperatur zwischen 60 °C und dem Siedepunkt des Lösungsmittels 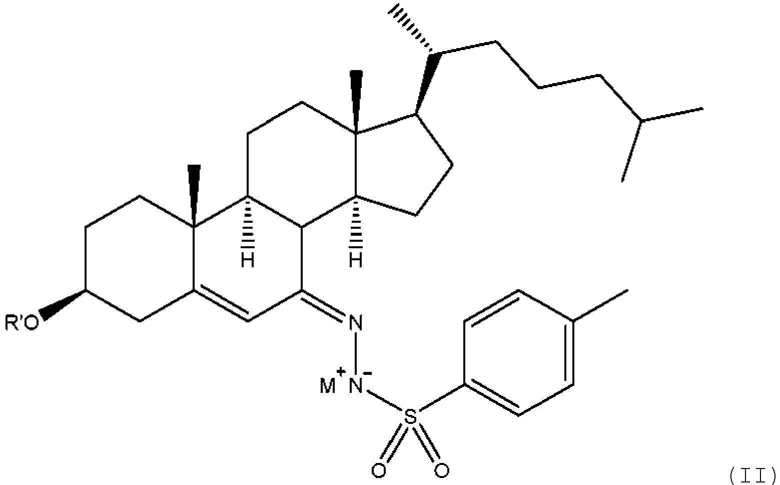 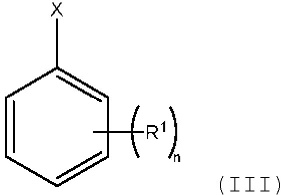 **dadurch gekennzeichnet, dass**
R' für eine OH-Schutzgruppe steht;
R für H oder R' steht;
M für ein Alkalimetallatom, vorzugsweise Li, steht;
X entweder für ein Halogenatom, vorzugsweise Cl, oder für eine C₁₋₆-Alkoxygruppe steht;
R¹ für eine C₁₋₆-Alkylgruppe steht;
n = 0 oder 1 oder 2 oder 3, vorzugsweise 0;
mit der Maßgabe, dass dann, wenn R für H steht,
das Verfahren ferner einen Schritt z) umfasst, der nach Schritt a) stattfindet:
z) Entschützen der geschützten Hydroxylgruppe der Verbindung der Formel (I-A) 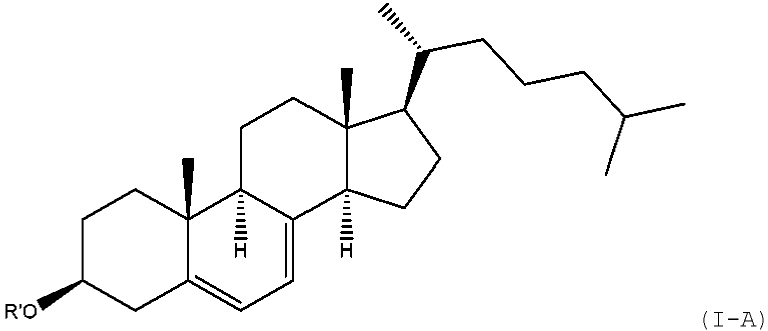
unter Bildung der Verbindung der Formel (I).

2. Verfahren nach Anspruch 2, **dadurch gekennzeichnet, dass** die Verbindung der Formel (II) durch Salzbildung einer Verbindung der Formel (IV) 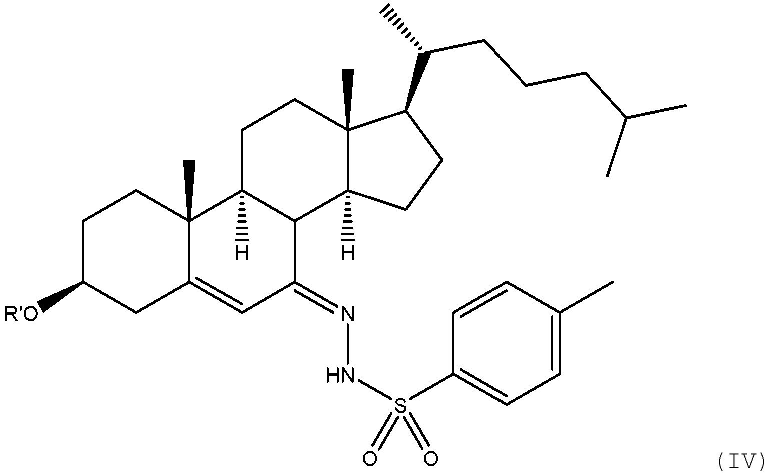 und einer starken Base, die ein Alkalimetall umfasst, insbesondere eines Alkalimetallalkoholats, Alkalimetallamids, Alkalimetallhydrids oder komplexen Alkalimetallhydrids, vorzugsweise mit NaNH₂ oder LiNH₂, hergestellt wird.

3. Verfahren nach Anspruch 1 oder 2, **dadurch gekennzeichnet, dass** das Salz der Formel (II) in Schritt a) während eines Zeitraums zwischen 1 und 120 Minuten, vorzugsweise zwischen 10 und 60 Minuten, zugegeben wird.

4. Verfahren nach einem der vorhergehenden Ansprüche, **dadurch gekennzeichnet, dass** die Temperatur des Lösungsmittels zwischen 60° und der Rückflusstemperatur des Lösungsmittels bei Umgebungsdruck, vorzugsweise zwischen 80 °C und 132 °C, liegt.

5. Verfahren nach einem der vorhergehenden Ansprüche 1-3 , **dadurch gekennzeichnet, dass** das Verfahren unter einem Druck von mehr als 1 bar durchgeführt wird.

6. Verfahren nach Anspruch 6, **dadurch gekennzeichnet, dass** die Temperatur des Lösungsmittels zwischen 60 und 200 °C liegt.

7. Verfahren nach einem der vorhergehenden Ansprüche, **dadurch gekennzeichnet, dass** R' für eine Acylgruppe, vorzugsweise eine Acetyl- oder Benzoylgruppe, insbesondere eine Acetylgruppe, steht.

## Revendications

1. Procédé de fabrication d'un composé de formule (I) 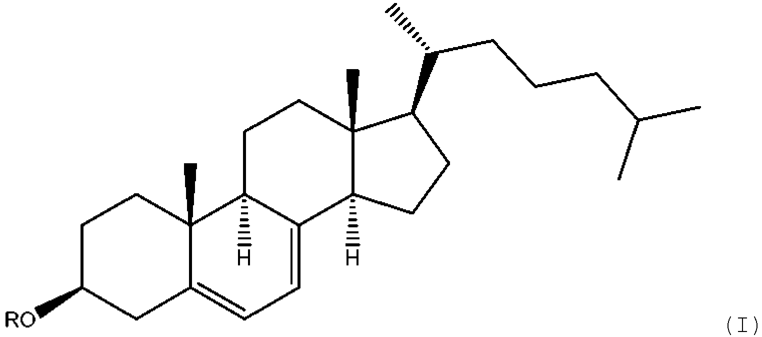 comprenant l'étape
a) ajout d'un sel de la formule (II) à un solvant de la formule (III) à une température comprise entre 60 °C et le point d'ébullition dudit solvant 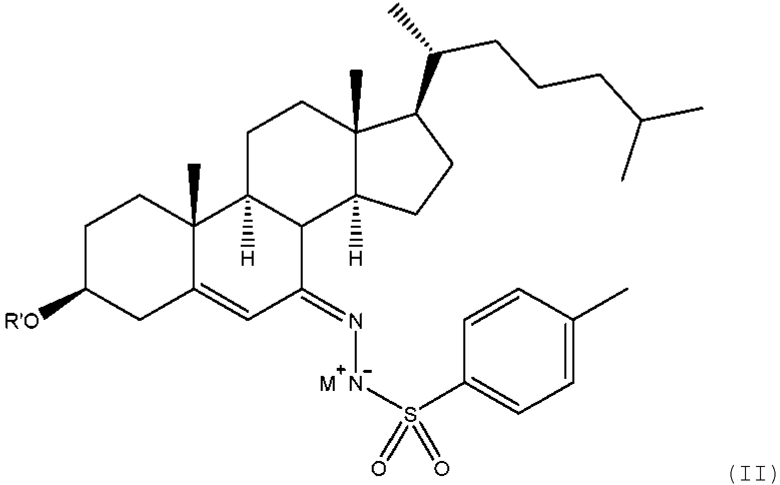 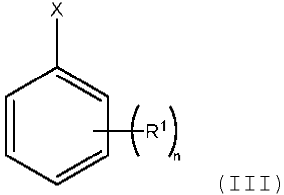 **caractérisé en ce que**
R' représente un groupe protecteur d'OH ;
R représente H ou R' ;
M est un atome de métal alcalin, préférablement Li ;
X représente soit un atome d'halogène, préférablement Cl, soit un groupe C₁₋₆-alcoxy ;
R¹ représente un groupe C₁₋₆-alkyle ;
n = 0 ou 1 ou 2 ou 3, préférablement 0 ;
à la condition que si R représente H,
le procédé comprenne en outre une étape z) qui ait lieu après l'étape a)
z) déprotection du groupe hydroxyle protégé du composé de la formule (I-A) 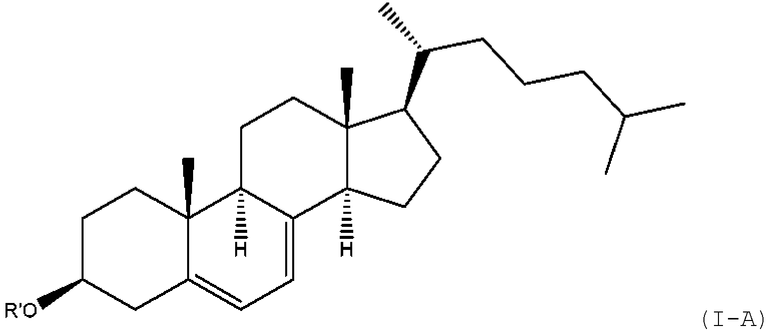
pour former le composé de la formule (I).

2. Procédé selon la revendication 2 **caractérisé en ce que** le composé de la formule (II) est préparé par formation d'un sel d'un composé de la formule (IV) 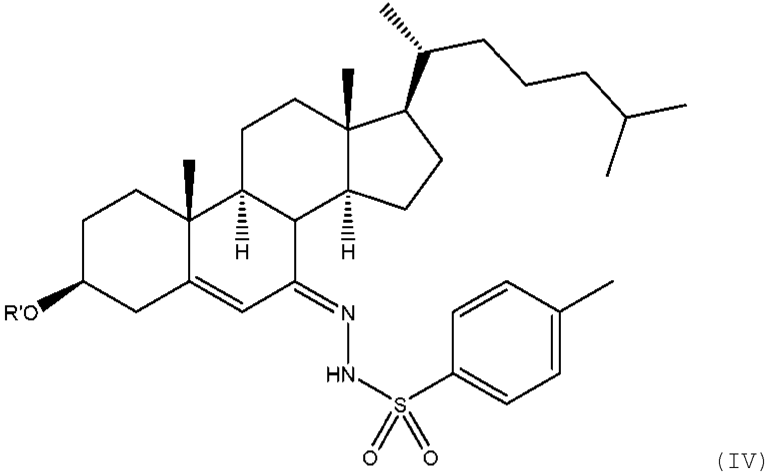 et une base forte comprenant un métal alcalin, particulièrement un alcoolate, un amide, un hydrure ou un hydrure complexe de métal alcalin, préférablement par NaNH₂ ou LiNH₂.

3. Procédé selon la revendication 1 ou 2 **caractérisé en ce que** le sel de la formule (II) est ajouté dans l'étape a) pendant une période de temps comprise entre 1 et 120 minutes, préférablement entre 10 et 60 minutes.

4. Procédé selon l'une quelconque des revendications précédentes **caractérisé en ce que** la température du solvant est comprise entre 60° et la température de reflux dudit solvant à pression ambiante, préférablement entre 80 °C et 132 °C.

5. Procédé selon l'une quelconque des revendications précédentes 1 à 3 **caractérisé en ce que** le procédé est réalisé sous une pression plus grande que 1 bar.

6. Procédé selon la revendication 6 **caractérisé en ce que** la température du solvant est comprise entre 60° et 200 °C.

7. Procédé selon l'une quelconque des revendications précédentes **caractérisé en ce que** R' représente un groupe acyle, préférablement un groupe acétyle ou benzoyle, particulièrement un groupe acétyle.
